# EUROPEAN PATENT SPECIFICATION

(11) **EP 4 356 934 B1**
(45) Date of publication and mention of the grant of the patent: **20.05.2026**
(21) Application number: 22824891.0
(22) Date of filing: 08.06.2022
(51) Int. Cl.: A61L 2/03, C12Q 1/02, G01N 27/416, A61C 19/06, A61C 8/00

(54) **METHOD FOR ADJUSTING ACTIVITY OF BIOFILM**
VERFAHREN ZUR EINSTELLUNG DER AKTIVITÄT EINES BIOFILMS
PROCÉDÉ D'AJUSTEMENT DE L'ACTIVITÉ D'UN BIOFILM

(30) Priority: 16.06.2021 JP 2021099812
(43) Date of publication of application: 24.04.2024
(73) Proprietor: National Institute for Materials Science, Tsukuba-shi, Ibaraki 305-0047 (JP)
(72) Inventor: OKAMOTO, Akihiro, Tsukuba-shi, Ibaraki 305-0047 (JP); MIRAN, Waheed, Tsukuba-shi, Ibaraki 305-0047 (JP)
(74) Representative: J A Kemp LLP
(86) International application number: PCT/JP2022/023139
(87) International publication number: WO 2022/264900

(56) References cited:
- JP-A- 2005 535 095
- JP-A- 2006 242 933
- JP-A- 2008 521 507
- JP-A- 2020 014 418
- KR-A- 20200 132 854
- US-A1- 2015 076 000

## Description

### Technical Field

The present invention relates to a biofilm activity adjusting method.

### Background Art

Metal implants have been used not only in dentistry and orthopedics fields but also in treatments of many diseases. However, bacteria grow on surfaces of metal implants to form a biofilm, therefore a patient's infection risk is increased, and once an infection occurs, measures such as removal of the metal implant are required in some cases.

As a method for controlling bacteria on surfaces of such metal implants, particularly for controlling a biofilm activity, PTL 1 describes "a method for providing a cathodic voltage controlled electric stimulation, including: providing a subject as a working electrode; performing a polarization scanning on the subject; autonomously detecting a hydrogen generation reaction threshold from the polarization scanning using a processor to determine a cathode voltage to be applied to the subject; and applying the determined cathode voltage to the subject".

### Citation List

### Patent Literature

PTL 1: International Publication No. WO 2020/247422
PTL 2: US 2015/076000 A1

### Summary of Invention

### Technical Problem

In the method described in PTL 1, a value of a response current in response to application of the cathode voltage to the subject was small, a biofilm activity suppressing effect was insufficient, and furthermore it was difficult to evaluate the progress and the degree of the activity suppression depending on the response current.

Thus, an object of the present invention is to provide a biofilm activity adjusting method that exhibits an excellent activity adjusting effect on the biofilm. Solution to Problem

As a result of intensive studies for solving the above problems, the present inventors have found that the above problems can be solved by the following configurations.
[1] A biofilm activity adjusting method, including: bringing a medium containing 2-hydroxy-1,4-naphthoquinone and water into contact with a conductor having a bacterium-derived biofilm; and adjusting the biofilm activity by applying, to the conductor, a first electric potential of higher than a lower limit value of an electric potential window and -0.4 V or lower with respect to a silver/silver chloride electrode reference.
[2] The biofilm activity adjusting method according to [1], in which the bacterium is a Gram-negative bacillus.
[3] The biofilm activity adjusting method according to [2], in which the Gram-negative bacillus belongs to Enterobacteriaceae.
[4] The biofilm activity adjusting method according to [3], in which the bacterium belonging to Enterobacteriaceae is at least one bacterium selected from a group consisting of genus Klebsiella, genus Enterobacter, genus Escherichia, genus Salmonella, genus Serratia, genus Shigella, and genus Yersinia.
[5] The biofilm activity adjusting method according to any one of [1] to [4], in which the conductor is made of a carbon material.
[6] The biofilm activity adjusting method according to any one of [1] to [4], including: measuring a current value after the application of the first electric potential; and providing information for determining a change in the biofilm activity by comparing the current value with a reference value.
[7] The biofilm activity adjusting method according to [6], in which the current value is a current density measured after elapse of a predetermined time from the application of the first electric potential.
[8] The biofilm activity adjusting method according to [7], in which the reference value is a current density measured after elapse of a predetermined time without application of the first electric potential.
[9] The biofilm activity adjusting method according to [8], in which the information is a difference between the current density measured after elapse of a predetermined time from the application of the first electric potential and the current density measured after elapse of a predetermined time without application of the first electric potential.
[10] The biofilm activity adjusting method according to any one of [1] to [9], in which the above conductor is a medical appliance to be implanted in a living body and a medical appliance to be placed beyond or in direct contact with a mucous membrane of a living body.
[11] The biofilm activity adjusting method according to any one of [1] to [9], including: bringing the bacterium, the conductor, and the medium into contact with each other; and applying, to the conductor, a second electric potential of higher than -0.4 V with respect to the silver/silver chloride electrode reference and lower than an upper limit value of the electric potential window to form the biofilm on the conductor.
[12] The biofilm activity adjusting method according to [11], in which the conductor is an electrode.
[13] The biofilm activity adjusting method according to [12], in which the second electric potential is 0 to 0.8 V with respect to the silver/silver chloride electrode reference.
[14] The biofilm activity adjusting method according to any one of [11] to [13], including: removing the medium after the formation of the biofilm; and replacing the medium with a new liquid medium containing no bacterium.
[15] The biofilm activity adjusting method according to [14], including applying the second electric potential to the conductor after the replacement; and providing, if an electric current generation is confirmed, information for determining formation of the biofilm on the conductor.

### Advantageous Effects of Invention

According to the present invention, a biofilm activity adjusting method that exhibits an excellent activity adjusting effect on a biofilm can be provided.

The biofilm activity adjusting method according to the present invention includes: bringing a medium containing 2-hydroxy-1,4-naphthoquinone (hereinafter, also referred to as "HNQ") and water into contact with a conductor having a bacterium-derived biofilm; and adjusting the biofilm activity by applying, to the conductor, a first electric potential of higher than a lower limit value of an electric potential window and lower than -0.4 V with respect to a silver/silver chloride electrode reference.

The HNQ as an electron mediator enhances the efficiency of electron transfer to the electrode. As a result, the electrons can be smoothly transferred between the bacteria and the electrode, resulting in a superior biofilm activity adjusting function.

When the biofilm formed on the conductor is derived from a Gram-negative bacillus, the electron transfer by the HNQ becomes more efficient, resulting in a superior biofilm activity adjusting function. This tendency is more remarkable when the Gram-negative bacillus belongs to Enterobacteriaceae and is furthermore at least one selected from a group consisting of genus Klebsiella, genus Enterobacter, genus Escherichia, genus Salmonella, genus Serratia, genus Shigella, and genus Yersinia.

The above bacterium is considered to be a causative bacterium of hospital-acquired infection. Use of the biofilm activity adjusting method according to the present invention makes it possible to adjust the activity of the biofilm derived from the causative bacterial colonies without using any antimicrobial agent or with a reduced amount of antimicrobial agents. Typically, the method can reduce the activity. Thus, the method can contribute to suppression of new drug-resistant bacteria generations.

If the conductor is made of a carbon material, the electron transfer by the HNQ becomes more efficient, resulting in a superior biofilm activity adjusting function.

If the biofilm activity adjusting method according to the present invention includes: measuring a current value after the application of the first electric potential; and providing information for determining a change in the biofilm activity by comparing the current value with a reference value, the change in the biofilm activity can be on-site and immediately determined.

Conventionally, determination of changes in the biofilm activity has required electromicroscopic observation of the biofilm on the electrode surface. However, in the present invention, the electron transfer becomes more efficient by using a liquid medium containing the HNQ, to obtain a sufficient response current, so that the biofilm activity can be evaluated more accurately, quickly, and nondestructively.

If the current value is a current density, the biofilm activity can be evaluated more accurately.

If the above reference value is a current density measured after elapse of a predetermined time without application of the first electric potential, the reference value is significant for a so-called control experiment, and the biofilm activity adjusting effect can be quantitatively evaluated.

If the conductor is a medical appliance to be implanted in a living body (e.g., various implants such as dental implants), and a medical appliance to be placed beyond or in direct contact with a mucous membrane of a living body (e.g., forceps, endoscopes, etc.), the activity can be adjusted (typically deactivated) even for a biofilm generated in small parts where mechanical cleaning is inaccessible.

If the biofilm activity adjusting method according to the present invention includes bringing the bacteria, the conductor, and the liquid medium into contact with each other, and applying, to the conductor, the second electric potential of -0.4 V or higher with respect to the silver/silver chloride electrode reference and lower than the upper limit value of the electric potential window to form a biofilm on the conductor, the method can be desirably applied for biofilm studies or the like in that a series of processes from formation to deactivation of the biofilm can be observed.

In the above case, the conductor is preferably an electrode. Typical examples include a working electrode of a three-electrode electrochemical cell.

If the second electric potential is 0 to 0.8 V with respect to the silver/silver chloride electrode reference, biofilms are more likely to form.

If the biofilm activity adjusting method according to the present invention includes removing the medium after the formation of the biofilm and replacing the medium with a new medium containing no bacteria, an influence of the suspended bacteria on the electric current generation in the subsequent step can be decreased, and therefore the biofilm activity adjusting effect can be evaluated more accurately.

If the biofilm activity adjusting method according to the present invention includes providing information for determining formation of the biofilm on the conductor when electric current generation is confirmed by applying the second electric potential to the conductor after exchange of the medium, the biofilm activity adjusting effect can be evaluated more accurately because, the biofilm inactivation step can be performed after formation of the biofilm is confirmed.

### Brief Description of Drawings

[Fig. 1] Fig. 1 is a flow chart of a first embodiment of a biofilm activity adjusting method according to the present invention.
[Fig. 2] Fig. 2 is a flow chart of a second embodiment of the biofilm activity adjusting method according to the present invention.
[Fig. 3] Fig. 3 is a flow chart (former half) of a third embodiment of the biofilm activity adjusting method according to the present invention.
[Fig. 4] Fig. 4 is a time-current curve comparing electric currents generated by a plurality of electron mediators.
[Fig. 5] Fig. 5 is a time-current curve indicating electric currents generated in a case that an electric potential of a working electrode is set to +0.2 V (vs. Ag/AgCl) in a medium containing 100 µM of HNQ as an electron mediator.
[Fig. 6] Fig. 6 is a time-current curve in a case that the medium was discarded, the electrode was washed, then a new medium (of which the composition is the same as of the prior medium except that the new medium does not contain Klebsiella pneumoniae) was added, and the electric potential of the working electrode is set to +0.2 V (vs. Ag/AgCl).
[Fig. 7] Fig. 7 is a schematic diagram of a conductor and bacteria constituting the biofilm.
[Fig. 8] Fig. 8 is a time-current curve indicating a change in a generated electric current in a case that a negative electric potential is applied to the working electrode having the biofilm.

### Description of Embodiments

The present invention will be explained below in detail.

Although the explanation of the constituent elements described below is based on representative embodiments of the present invention in some cases, the present invention is not limited to such embodiments.

In this specification, a numerical value range expressed by "to" means a range including the numerical values described before and after "to" as lower and upper limit values.

### [First Embodiment of Biofilm Activity Adjusting Method]

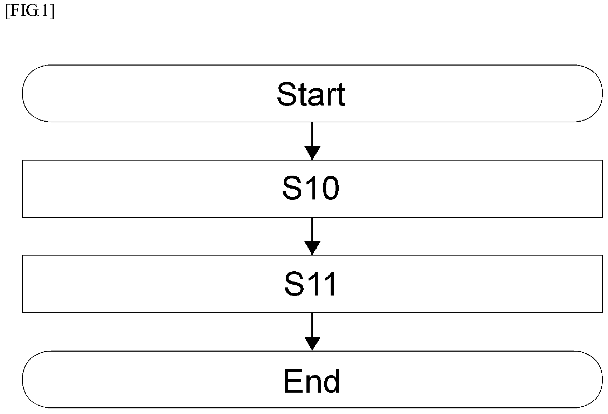

The first embodiment of the biofilm activity adjusting method according to the present invention (hereinafter, also referred to as "Embodiment 1") will be explained with reference to figures. Fig. 1 is a flow chart of Embodiment 1.

First, a medium is brought into contact with a conductor having a bacterium-derived biofilm (step S10).

Herein, the medium contains 2-hydroxy-1,4-naphthoquinone (HNQ) and water.

A content of the HNQ in the medium is not particularly limited, and is preferably 1 to 1000 µmol/L, for example.

The medium may contain other components as long as it contains the HNQ and water. Examples of other components include an electron source compound, an electron mediator other than the HNQ, a buffer agent, and a coagulant.

The electron source compound is used for bacterial metabolism, and examples of the electron source compound include, but are not limited to, organic compounds (amino acids, sugars, organic acids, etc.).

As the electron mediator other than the HNQ, it is possible to use a known compound such as 1-hydroxyphenazine, anthraquinone-1,5-disulfonic acid (disodium salt), anthraquinone-1-sulfonic acid (sodium salt), flavin mononucleotide, and riboflavin.

Examples of the buffer agent include, but are not particularly limited to, borate, bicarbonate, Tris-HCl, citrate, phosphate, succinate, phosphate, and acetate.

Examples of the coagulant include agarose, gelatin, and agar. Of which, agar is preferable.

As the medium, it is possible to use a liquid or solid culture medium containing each component described above, or a medium with a predetermined amount of HNQ added to a culture medium prepared by a prescribed method.

The material, shape, and size of the conductor is not particularly limited as long as the conductor can transfer and receive electrons to and from the biofilm.

In particular, the conductor is preferably made of a carbon material from the viewpoint of allowing detection of electric current generation with higher sensitivity in a combination of the conductor with the HNQ and the bacteria belonging to Enterobacteriaceae. The phrase "the conductor is made of a carbon material" means that at least a face in contact with the biofilm is made of a carbon material.

The carbon material is not particularly limited and may be any carbon electrode used for electrochemical measurement and the like. Examples of the carbon material include amorphous carbon, graphite, carbon nanotube, and mixtures thereof.

Examples of the conductor include a medical appliance that is implanted in a living body (medical implant, etc.), a medical appliance that is placed beyond a mucous membrane of a living body (forceps, etc.), and a medical appliance that is placed in direct contact with a mucous membrane of a living body (endoscope, etc.). However, the claimed method does not comprise the treatment of implants located in a human or animal body as the method is limited to non-therapeutic applications.

If such a conductor is used, Embodiment 1 can be utilized as a method for cleaning and washing medical appliances and medical implants.

Although the targeted bacteria are not particularly limited, Gram-negative bacilli are favorable.

In particular, if the Gram-negative bacilli belong to Enterobacteriaceae, the effect of the present invention is improved.

Examples of the bacteria belonging to Enterobacteriaceae include genus Klebsiella, genus Enterobacter, genus Escherichia, genus Salmonella, genus Serratia, genus Shigella, and genus Yersinia.

The method for bringing the medium into contact with the conductor having the biofilm is not particularly limited. For example, if the medium is liquid, a method in which a conductor is immersed in the medium, or a method in which the medium is dripped onto the conductor can be used.

If the medium is solid, a method in which the medium is brought into direct contact with the conductor under pressure or without pressure can be used. The phrase "brought into direct contact with" typically means that, if the electrode is a flat plate, the face on which the biofilm is formed is brought into contact with the medium.

Subsequently, the first electric potential of higher than a lower limit value of an electric potential window and -0.4 V or lower with respect to a silver/silver chloride electrode reference is applied to the conductor to adjust the biofilm activity (step S11). Preferably, this step is performed under anaerobic atmosphere.

The lower limit value of the electric potential window depends on the composition of the medium, the pH of the medium, the material of the electrode, or the like, and is obvious to those skilled in the art.

The lower limit value of the electric potential in this method is not particularly limited as long as it is higher than the lower limit value of the electric potential window. The lower limit value of the electric potential is preferably -1.2 V or higher, more preferably -1.1 V or higher, further preferably -1.0 V or higher with respect to the silver/silver chloride electrode reference.

The method for applying the first electric potential to the conductor is not particularly limited. Typically, it is possible to use a method in which a counter electrode and a reference electrode are brought into contact with the medium, the conductor already described is used as the reference electrode, and the electrode set thereof (working electrode, counter electrode, and reference electrode) is connected to a potentiostat, a predetermined electric potential is applied to the working electrode while controlling the set.

The materials for the counter electrode and the reference electrode are not particularly limited. Any known counter electrode or reference electrode for electrochemical measurement or the like can be used. Preferably, the reference electrode is a silver/silver chloride electrode.

The medium already described may be liquid or solid. If the medium is liquid, the counter electrode and the reference electrode should be immersed in the medium. In a configuration, the medium may be dripped onto the electrode set (conductors: working electrode, counter electrode, and reference electrode) fabricated by a printed electronics or the like, such that they are bought into contact with each other.

If the medium is solid, the counter electrode and reference electrode should be brought into direct contact with the medium in the same manner as for the above conductor.

As described in Examples below, when a negative electric potential is applied to the conductor (working electrode), electrons are prevented from flowing from the bacteria to the conductor.

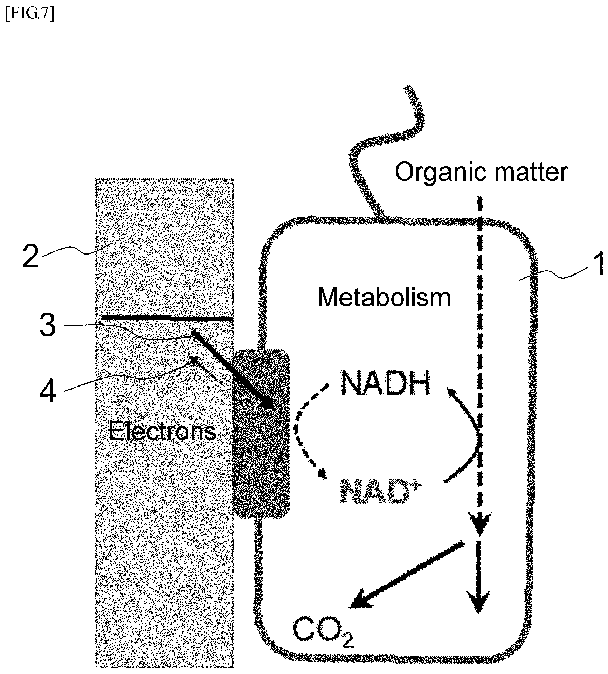

Fig. 7 is a schematic diagram of the conductor according to the present invention and the bacteria constituting the biofilm. Bacterial cells 1 form a biofilm and adhere to the conductor 2. In the process where the bacteria decompose organic matters to acquire energy (particularly under anaerobic condition), reduced nicotinamide adenine dinucleotide (NADH) is produced from oxidized nicotinamide adenine dinucleotide (NAD⁺) used to decompose the organic matters, electrons are transferred from NADH to the conductor as an electron acceptor through an electron transfer system (arrow 4), then electrons return to NAD⁺ and are again used for acquiring energy.

In contrast, when the first electric potential is applied to the conductor 2, the transfer of electrons from the bacterial cells 1 to the conductor 2 (arrow 4) is restricted. This prevents the cells from acquiring energy, the cell's activity is suppressed. Furthermore, when electrons are transferred from the conductor 2 to the bacterial cells 1 (arrow 3), the activity of the bacterial cells 1 is further suppressed.

This method makes it possible to adjust (suppress) the biofilm activity simply by applying a predetermined electric potential to the conductor without using any antimicrobial agent or the like.

This method can be used to study biofilms, and furthermore, when the conductor is a medical appliance, a medical implant, or the like, the biofilm can be deactivated even if the conductor is hard to mechanically clean due to its complicated shape, or the like. The method can be expected to exhibit the effect of preventing bacterial infection.

### [Second Embodiment of Biofilm Activity Adjusting Method]

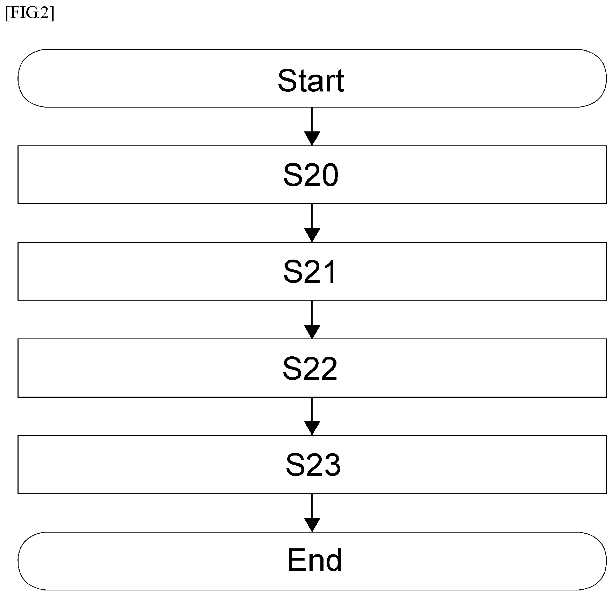

The second embodiment of the biofilm activity adjusting method according to the present invention (hereinafter, also referred to as "Embodiment 2") will be explained with reference to figures. Fig. 2 is a flow chart of Embodiment 2.

First, the medium is brought into contact with the conductor having the bacterium-derived biofilm (step S20).

Next, the first electric potential of higher than the lower limit value of the electric potential window and -0.4 V or lower with respect to the silver/silver chloride electrode reference is applied to the conductor to adjust the biofilm activity (step S21). It is preferable that this step is performed under anaerobic atmosphere.

The step S20 and step S21 are the same as the step S10 and step S11 respectively in Embodiment 1, and the suitable configurations are also the same as in Example 1, and therefore explanation of the step S20 and step S21 is omitted.

Subsequently, the current value is measured (step S22). The method for measuring the current value is not particularly limited. Typically, the current value can be measured using a potentiostat that is connected to the electrode set when applying the first electric potential to the conductor, as explained in Embodiment 1. The electric current measured in this case may be a current density.

Also, typically, a time-current (current density) curve is measured.

Then, the obtained current values are compared to a reference value to provide information for determining a change in the biofilm activity (step S23).

As is clear from Examples described below, an increase in the activity of the biofilm (i.e. bacterial colony present in the inside) is detected as an electron transfer to the conductor (working electrode), i.e. as a current (density) value. The current value increases with time.

On the other hand, as the biofilm activity decreases, the current value decreases. This is caused by hindrance or decrease of the electron transfer to the conductor (working electrode).

The current value is provided as information for determining such a change in the biofilm activity. Examples of the difference between the measured current value and the reference value include, but are not particularly limited to, a difference between a current (density) value measured after elapse of a predetermined time from application of an electric potential and a reference value, a difference between a maximum current (density) value within a predetermined time from application of an electric potential and a reference value, a difference between an increase in a current value within a predetermined time and a reference value, and a difference between an integral value within a predetermined time in a time-current (density) curve and a reference value.

In particular, a case where the reference value is a current (density) value measured without application of the first electric potential is favorable in that more accurate information can be provided. The phrase "measured without application of the first electric potential" means that the reference value was measured under the same condition as for the generated electric current except that the first electric potential was not applied.

In particular, from the viewpoint of providing information for more easily determining the change in the biofilm activity, it is preferable that the measured current value is a current density measured after elapse of a predetermined time from application of the first electric potential, and the reference value is a current density measured without application of the first electric potential (other conditions are the same).

### [Third Embodiment of Biofilm Activity Adjusting Method]

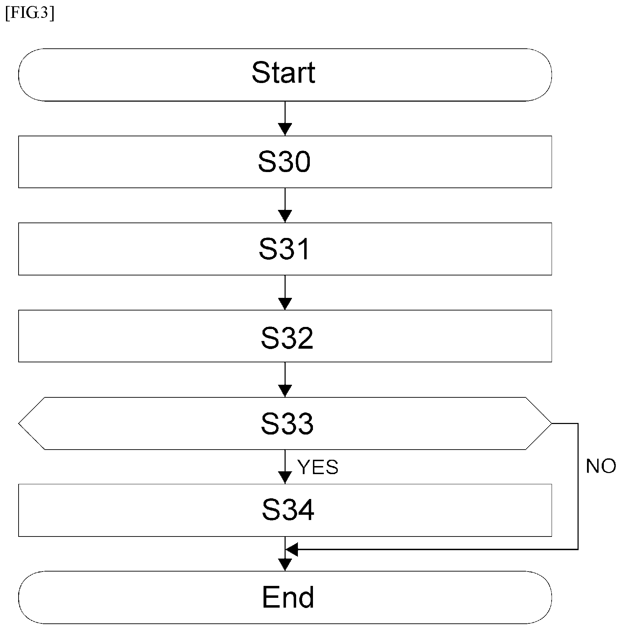

The third embodiment of the biofilm activity adjusting method according to the present invention (hereinafter, also referred to as "Embodiment 3") will be explained with reference to figures. Fig. 3 is a flow chart of the former half of the third embodiment. The "former half" means that a conductor having the biofilm can be obtained after the flow of Fig. 3, and therefore the biofilm activity can be adjusted according to the procedure of Embodiment 1 or Embodiment 2 using the conductor.

In other words, for the "latter half" flow after the flow of Fig. 3, the flow of Embodiment 1 or Embodiment 2 can be adopted without modification, and therefore explanation of this part is omitted.

Returning to Fig. 3, first, the bacteria, the conductor, and the medium are brought into contact with each other, and the second electric potential of higher than -0.4 V with respect to the silver/silver chloride electrode reference and lower than the upper limit value of the electric potential window is applied to the conductor to form a biofilm on the conductor (step S30).

Preferably, this step is performed under anaerobic atmosphere.

Since the bacteria, conductor, and medium for use, as well as the suitable configurations are the same as those described in Embodiment 1, explanation thereof is omitted. Also, explanation of the method for applying the electric potential is omitted because the same method as described in Embodiment 1 for applying the first electric potential to the conductor can be used.

When the second electric potential is applied to the conductor in contact with the medium containing the bacteria, electrons are smoothly transferred from the NADH to the conductor 2 (arrow 4 in Fig. 7), so that formation of the biofilm is enhanced.

The second electric potential in this case is not particularly limited as long as the second electric potential is higher than -0.4 V (vs. Ag/AgCl) and lower than the upper limit value of the electric potential window. Generally, the second electric potential is preferably 0 to 0.8 V (vs. Ag/AgCl).

Subsequently, the medium is replaced with a new bacterium-free medium (step S31).

In this step, since the suspended bacteria in the medium can be removed, the measured value in the subsequent step can be more accurate. In particular, when the medium is a liquid medium, the effect of removing the suspended bacteria is remarkable.

The biofilm activity adjusting method according to this embodiment need not include this step. If the method does not include this step, it is preferable to omit the subsequent steps S32 to S34.

Subsequently, the second electric potential is applied to the conductor (step S32). Then, the presence or absence of the electric current generation is confirmed (step S33).

In these steps, the suspended bacteria have been removed from the medium by step S31, and therefore, if the electric current generation can be confirmed (step S33: YES), the electric current is likely to be attributed to the biofilm formed on the conductor and is provided as information for determining formation of the biofilm on the conductor (step S34).

The information provided include a current (density) value measured after elapse of a predetermined time from the application of the second electric current, and the like. In addition to the aforementioned information, an increase in the current (density) value within a predetermined period of time, and the like may be included.

The biofilm activity adjusting method according to this embodiment has advantageous that the process of forming the biofilm on the conductor can be observed depending on the generated electric current, and the biofilm activity can be evaluated more accurately depending on the electric current generated in the subsequent step (Embodiment 1 or Embodiment 2).

The biofilm activity adjusting method according to this embodiment makes it possible to observe a series of bacterial activities from biofilm formation to inactivation depending on the generated electric current. Thereby, the method can be suitably used for studying the bacterial action of forming the biofilm, or the like.

Conventionally, such a method has been difficult to practically use due to small generated electric current and difficult detection, but an inventors' new discovery that the generated electric current is sufficiently increased by using the HNQ has completed the present invention.

### EXAMPLES

The biofilm activity adjusting method according to the embodiment of the present invention will be explained below on the basis of experimental data. Note that the following experimental data are intended to explain a configuration of the method and are not intended to limit the experimental conditions and the like of the method.

The experiment was performed using a three-electrode electrochemical cell having carbon electrodes as the working electrode and counter electrode, and a silver/silver chloride electrode as the reference electrode.

First, as an electron source, a liquid culture medium (defined medium) containing 10 mM of glucose and 100 µM of electron mediator (see below) was added to the cell described above, to which Klebsiella pneumoniae (optical density (OD): 0.1) was inoculated, then an electric potential of the working electrode was set to +0.2 V (vs. Ag/AgCl) at 37°C, and the culture medium was incubated under anaerobic condition for 15 hours.

### <Electron Mediator for Use>

HNQ: 2-hydroxy-1,4-naphthoquinone
1-Phenazine: 1-hydroxyphenazine
AQDS: anthraquinone-1,5-disulfonate
AQS: anthraquinone-1-sulfonate
FMN: flavin mono nucleotide
RF: ribobflavin
W/O mediator: no electron mediator

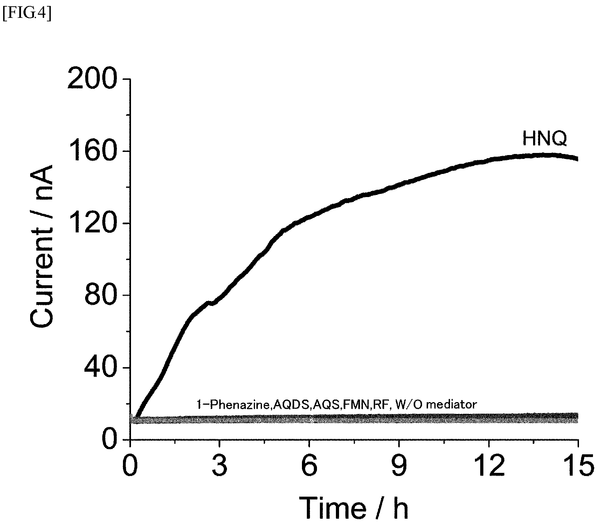

Fig. 4 is a time-current curve. The results in Fig. 4 revealed that a large electric current could be generated by using the HNQ.

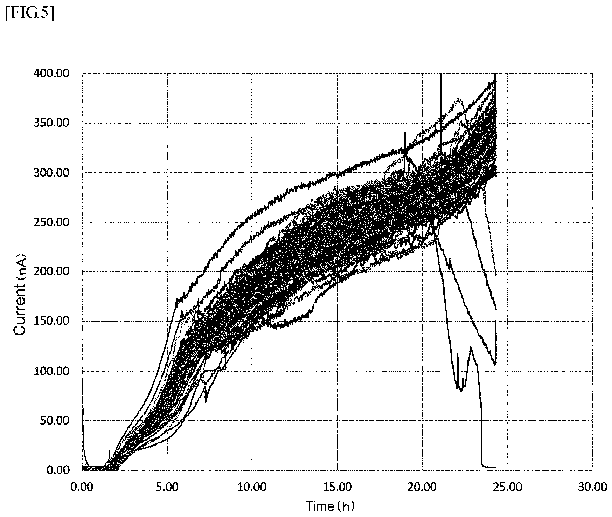

Fig. 5 is a time-current curve indicating electric currents generated in a case that an electric potential of a working electrode is +0.2 V (vs. Ag/AgCl) in a medium containing 100 µM of HNQ as an electron mediator.

The experiment was performed under anaerobic environment using an electrochemical measurement plate with the aforementioned three electrodes printed on a bottom of each well of a 96-well plate.

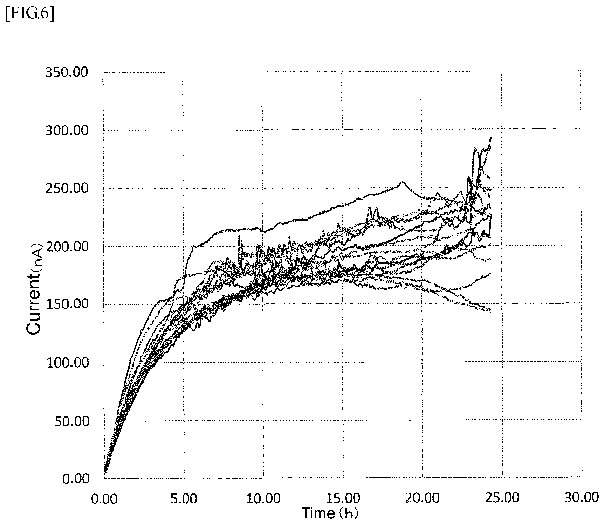

Fig. 6 is a time-current curve in a case that, after the test of Fig. 5, the medium was discarded, the electrode was washed, then a new medium (of which the composition is the same as of the medium of Fig. 5 except that the new medium does not contain Klebsiella pneumoniae) was added, and the electric potential of the working electrode was set to +0.2 V (vs. Ag/AgCl).

The results in Fig. 5 and Fig. 6 showed that the biofilm was formed on the working electrode and activated by application of a predetermined electric potential.

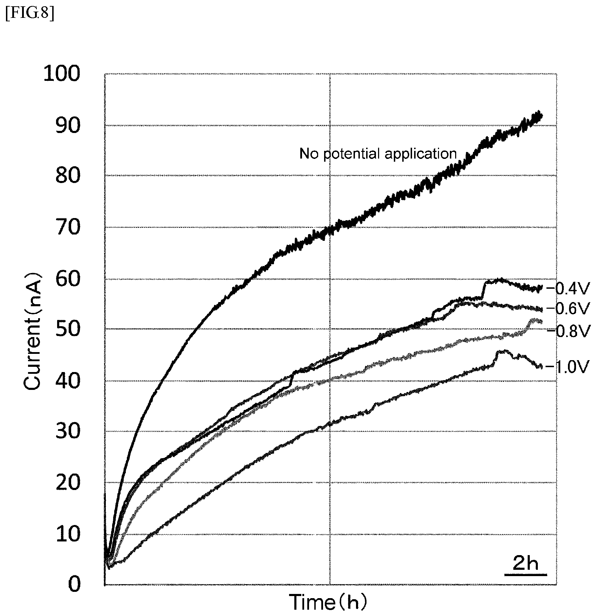

Fig. 8 is a time-current curve indicating a change in an electric current generated in a case that a negative electric potential is applied to the working electrode having the biofilm formed as above.

In Fig. 8, the description "No electric potential application" indicates an electric current generated in a case that, after the biofilm is formed, no electric potential is applied.

On the other hand, each of the descriptions "-0.4 V", "-0.6 V", "-0.8 V", and "-1.0 V" represents an electric current generated when the electric potential "-0.4 V", "-0.6 V", "-0.8 V", or "-1.0 V" respectively is applied to the working electrode.

The results in Fig. 8 showed that, when a prescribed electric potential was applied, the generated electric current decreased, the biofilm was deactivated.

### Industrial Applicability

The biofilm activity adjusting method according to the present invention can be used for washing and cleaning to prevent infections caused by medical appliances and medical implants. Also, the method can be used for studying biofilms.

## Claims

1. A non-therapeutic biofilm activity adjusting method, comprising:
bringing a medium containing 2-hydroxy-1,4-naphthoquinone and water into contact with a conductor having a bacterium-derived biofilm; and
adjusting the biofilm activity by applying, to the conductor, a first electric potential of higher than a lower limit value of an electric potential window and -0.4 V or lower with respect to a silver/silver chloride electrode reference.

2. The biofilm activity adjusting method according to claim 1, wherein the bacterium is a Gram-negative bacillus.

3. The biofilm activity adjusting method according to claim 2, wherein the Gram-negative bacillus belongs to Enterobacteriaceae.

4. The biofilm activity adjusting method according to claim 3, wherein the bacterium belonging to Enterobacteriaceae is at least one bacterium selected from a group consisting of genus Klebsiella, genus Enterobacter, genus Escherichia, genus Salmonella, genus Serratia, genus Shigella, and genus Yersinia.

5. The biofilm activity adjusting method according to any one of claims 1 to 4, wherein the conductor is made of a carbon material.

6. The biofilm activity adjusting method according to any one of claims 1 to 4, comprising:
measuring a current value after the application of the first electric potential; and
providing information for determining a change in the biofilm activity by comparing the current value with a reference value.

7. The biofilm activity adjusting method according to claim 6, wherein the current value is a current density measured after elapse of a predetermined time from the application of the first electric potential.

8. The biofilm activity adjusting method according to claim 7, wherein the reference value is a current density measured after elapse of a predetermined time without application of the first electric potential.

9. The biofilm activity adjusting method according to claim 8, wherein the information is a difference between the current density measured after elapse of a predetermined time from the application of the first electric potential and the current density measured after elapse of a predetermined time without application of the first electric potential.

10. The biofilm activity adjusting method according to any one of claims 1 to 9, wherein the above conductor is a medical appliance to be implanted in a living body and a medical appliance to be placed beyond or in direct contact with a mucous membrane of a living body.

11. The biofilm activity adjusting method according to any one of claims 1 to 9, comprising:
bringing the bacterium, the conductor, and the medium into contact with each other; and
applying, to the conductor, a second electric potential of higher than -0.4 V with respect to the silver/silver chloride electrode reference and lower than an upper limit value of the electric potential window to form the biofilm on the conductor.

12. The biofilm activity adjusting method according to claim 11, wherein the conductor is an electrode.

13. The biofilm activity adjusting method according to claim 12, wherein the second electric potential is 0 to 0.8 V with respect to the silver/silver chloride electrode reference.

14. The biofilm activity adjusting method according to any one of claims 11 to 13, comprising: removing the medium after the formation of the biofilm; and replacing the medium with a new liquid medium containing no bacterium.

15. The biofilm activity adjusting method according to claim 14, comprising: applying the second electric potential to the conductor after the replacement; and providing, if an electric current generation is confirmed, information for determining formation of the biofilm on the conductor.

## Patentansprüche

1. Nicht-therapeutisches Verfahren zur Regulierung der Biofilmaktivität, das umfasst:
Inkontaktbringen eines Mediums, das 2-Hydroxy-1,4-naphthochinon und Wasser enthält, mit einem Leiter, der einen aus Bakterien stammenden Biofilm aufweist; und
Regulieren der Biofilmaktivität durch Anlegen eines ersten elektrischen Potentials an den Leiter, das höher ist als ein unterer Grenzwert eines elektrischen Potentialfensters und -0,4 V oder niedriger in Bezug auf eine Silber/Silberchlorid-Referenzelektrode ist.

2. Verfahren zur Regulierung der Biofilmaktivität nach Anspruch 1, wobei das Bakterium ein gramnegativer Bazillus ist.

3. Verfahren zur Regulierung der Biofilmaktivität nach Anspruch 2, wobei der gramnegative Bazillus zur Familie der Enterobacteriaceae gehört.

4. Verfahren zur Regulierung der Biofilmaktivität nach Anspruch 3, wobei das zu den Enterobacteriaceae gehörende Bakterium mindestens ein Bakterium ist, ausgewählt aus einer Gruppe bestehend aus der Gattung Klebsiella, der Gattung Enterobacter, der Gattung Escherichia, der Gattung Salmonella, der Gattung Serratia, der Gattung Shigella und der Gattung Yersinia.

5. Verfahren zur Regulierung der Biofilmaktivität gemäß einem der Ansprüche 1 bis 4, wobei der Leiter aus einem Kohlenstoffmaterial besteht.

6. Verfahren zur Regulierung der Biofilmaktivität gemäß einem der Ansprüche 1 bis 4, das umfasst:
Messen eines Stromwerts nach Anlegen des ersten elektrischen Potentials; und
Bereitstellen von Informationen zur Bestimmung einer Änderung der Biofilmaktivität durch Vergleichen des Stromwerts mit einem Referenzwert.

7. Verfahren zur Regulierung der Biofilmaktivität nach Anspruch 6, wobei der Stromwert eine Stromdichte ist, die nach Ablauf einer vorbestimmten Zeit ab dem Anlegen des ersten elektrischen Potentials gemessen wird.

8. Verfahren zur Regulierung der Biofilmaktivität nach Anspruch 7, wobei der Referenzwert eine Stromdichte ist, die nach Ablauf einer vorbestimmten Zeit ohne Anlegen des ersten elektrischen Potentials gemessen wird.

9. Verfahren zur Regulierung der Biofilmaktivität nach Anspruch 8, wobei die Informationen eine Differenz sind zwischen der Stromdichte, die nach Ablauf einer vorbestimmten Zeit ab dem Anlegen des ersten elektrischen Potentials gemessen wird, und der Stromdichte, die nach Ablauf einer vorbestimmten Zeit ohne Anlegen des ersten elektrischen Potentials gemessen wird.

10. Verfahren zur Regulierung der Biofilmaktivität gemäß einem der Ansprüche 1 bis 9, wobei der vorgenannte Leiter eine medizinische Vorrichtung ist, die in einen lebenden Körper implantiert werden soll, sowie eine medizinische Vorrichtung ist, die jenseits einer Schleimhaut eines lebenden Körpers oder in direktem Kontakt mit dieser angeordnet werden soll.

11. Verfahren zur Regulierung der Biofilmaktivität gemäß einem der Ansprüche 1 bis 9, das umfasst:
Inkontaktbringen des Bakteriums, des Leiters und des Mediums miteinander; und
Anlegen eines zweiten elektrischen Potentials an den Leiter,
das höher ist als -0,4 V in Bezug auf die Silber/Silberchlorid-Referenzelektrode und niedriger als ein oberer Grenzwert des elektrischen Potentialfensters ist, um den Biofilm auf dem Leiter zu bilden.

12. Verfahren zur Regulierung der Biofilmaktivität nach Anspruch 11, wobei der Leiter eine Elektrode ist.

13. Verfahren zur Regulierung der Biofilmaktivität nach Anspruch 12, wobei das zweite elektrische Potential 0 bis 0,8 V in Bezug auf die Silber/Silberchlorid-Referenzelektrode beträgt.

14. Verfahren zur Regulierung der Biofilmaktivität gemäß einem der Ansprüche 11 bis 13, das umfasst: Entfernen des Mediums nach der Bildung des Biofilms; und Ersetzen des Mediums durch ein neues flüssiges Medium, das keine Bakterien enthält.

15. Verfahren zur Regulierung der Biofilmaktivität nach Anspruch 14, das umfasst:
Anlegen des zweiten elektrischen Potentials an den Leiter nach dem Austausch; und
Bereitstellen, falls eine Stromerzeugung bestätigt wird, von Informationen zur Bestimmung der Bildung des Biofilms auf dem Leiter.

## Revendications

1. Procédé non thérapeutique de régulation de l'activité d'un biofilm, comprenant :
la mise en contact d'un milieu contenant de la 2-hydroxy-1,4-naphtoquinone et de l'eau avec un conducteur présentant un biofilm dérivé de bactéries, et
la régulation de l'activité d'un biofilm par application, au conducteur, d'un premier potentiel électrique supérieur à une valeur limite inférieure d'une fenêtre de potentiel électrique et inférieur ou égal à -0,4 V par rapport à une électrode de référence argent/chlorure d'argent.

2. Procédé de régulation de l'activité d'un biofilm selon la revendication 1, dans lequel la bactérie est un bacille Gram-négatif.

3. Procédé de régulation de l'activité d'un biofilm selon la revendication 2, dans lequel le bacille Gram-négatif appartient à la famille des entérobactériacées.

4. Procédé de régulation de l'activité d'un biofilm selon la revendication 3, dans lequel la bactérie appartenant à la famille des entérobactériacées est au moins une bactérie choisie dans un groupe constitué du genre Klebsiella, du genre Enterobacter, du genre Escherichia, du genre Salmonella, du genre Serratia, du genre Shigella et du genre Yersinia.

5. Procédé de régulation de l'activité d'un biofilm selon l'une quelconque des revendications 1 à 4, dans lequel le conducteur est constitué d'un matériau carboné.

6. Procédé de régulation de l'activité d'un biofilm selon l'une quelconque des revendications 1 à 4, comprenant :
la mesure d'une valeur du courant après l'application du premier potentiel électrique, et
la fourniture d'informations permettant de déterminer une variation de l'activité du biofilm par comparaison de ladite valeur du courant à une valeur de référence.

7. Procédé de régulation de l'activité d'un biofilm selon la revendication 6, dans lequel la valeur du courant est une densité de courant mesurée au terme d'une durée prédéterminée à compter de l'application du premier potentiel électrique.

8. Procédé de régulation de l'activité d'un biofilm selon la revendication 7, dans lequel la valeur de référence est une densité de courant mesurée au terme d'une durée prédéterminée sans application du premier potentiel électrique.

9. Procédé de régulation de l'activité d'un biofilm selon la revendication 8, dans lequel les informations correspondent à la différence entre la densité de courant mesurée au terme d'une durée prédéterminée à compter de l'application du premier potentiel électrique et la densité de courant mesurée au terme d'une durée prédéterminée sans application du premier potentiel électrique.

10. Procédé de régulation de l'activité d'un biofilm selon l'une quelconque des revendications 1 à 9, dans lequel le conducteur susmentionné est un dispositif médical destiné à être implanté dans un corps vivant et un dispositif médical destiné à être placé derrière ou en contact direct avec une membrane muqueuse d'un corps vivant.

11. Procédé de régulation de l'activité d'un biofilm selon l'une quelconque des revendications 1 à 9, comprenant :
la mise en contact de la bactérie, du conducteur et du milieu les uns avec les autres, et
l'application, au conducteur, d'un deuxième potentiel électrique supérieur à -0,4 V par rapport à l'électrode de référence argent/chlorure d'argent et inférieur à une valeur limite supérieure de la fenêtre de potentiel électrique afin de former le biofilm sur le conducteur.

12. Procédé de régulation de l'activité d'un biofilm selon la revendication 11, dans lequel le conducteur est une électrode.

13. Procédé de régulation de l'activité d'un biofilm selon la revendication 12, dans lequel le deuxième potentiel électrique est de 0 à 0,8 V par rapport à l'électrode de référence argent/chlorure d'argent.

14. Procédé de régulation de l'activité d'un biofilm selon l'une quelconque des revendications 11 à 13, comprenant : l'élimination du milieu après la formation du biofilm et le remplacement du milieu par un nouveau milieu liquide ne contenant aucune bactérie.

15. Procédé de régulation de l'activité d'un biofilm selon la revendication 14, comprenant : l'application du deuxième potentiel électrique au conducteur après le remplacement et la fourniture, si la génération d'un courant électrique est confirmée, d'informations permettant de déterminer la formation du biofilm sur le conducteur.
